# EUROPEAN PATENT APPLICATION

(11) **EP 4 582 535 A1**
(43) Date of publication of application: **09.07.2025**
(21) Application number: 24206616.5
(22) Date of filing: 15.10.2024
(51) Int. Cl.: C12N 5/078, A61K 38/18

(54) **METHOD FOR PREPARING EXOSOMES FROM HUMAN PLATELET**

(30) Priority: 05.01.2024 TW 113100664
(71) Applicant: Aventacell Biomedical Corp. Ltd., New Taipei City 241 (TW)
(72) Inventor: HUANG, Min-Chang, 241 New Taipei City (TW); LIN, Yee-Hsien, 241 New Taipei City (TW); LIN, Han-Tse, 241 New Taipei City (TW)
(74) Representative: Viering, Jentschura & Partner mbB Patent- und Rechtsanwälte

(57) **Abstract**

A method for preparing exosomes is provided. The method includes preparing a human platelet, treating the human platelet with a buffer to obtain a platelet solution, centrifuging the platelet solution, and collecting supernatant of the centrifuged platelet solution to obtain an exosome solution. The buffer is calcium ion buffer, phosphate buffered saline (PBS), tris(hydroxymethyl)aminomethane hydrochloride (Tris-HCl) buffer, or 2-[4-(2-hydroxyethyl)-1-piperazinyl]ethanesulfonic acid (HEPES) buffer.

## Description

### BACKGROUND

### Technical Field

The instant disclosure relates to a method for preparing exosomes, and particularly relates to a method for preparing exosomes from a platelet treated with a particular buffer.

### Related Art

Exosomes are a type of small endosomal-derived membrane microvesicles, which are a type of extracellular vesicles. Lipid bilayer vesicles of the exosomes carry various signaling factors such as nucleic acids, proteins, saccharides and lipids. The exosomes serve as carriers of these functional substances, are media for intercellular signal transmission, and can effectively control the expression of cellular genes and protein functions. Moreover, the function of the exosomes depends on the type of cell they derive from. The exosomes have multiple applications, and are related to various types of clinical research.

### SUMMARY

In some embodiments, a method for preparing exosomes includes providing a human platelet, treating the human platelet with a buffer to obtain a platelet solution, centrifuging the platelet solution, and collecting supernatant of the centrifuged platelet solution to obtain an exosome solution. The buffer is calcium ion buffer, phosphate buffered saline (PBS), tris(hydroxymethyl)aminomethane hydrochloride (Tris-HCl, pH 7.0 to 8.0) buffer or 2-[4-(2-hydroxyethyl)-1-piperazinyl]ethanesulfonic acid (HEPES) buffer.

In some embodiments, the calcium ion buffer includes phosphate buffered saline and at least one of calcium chloride, calcium carbonate, and calcium gluconate.

In some embodiments, a concentration of the aforementioned calcium chloride ranges from 2 mM to 20 mM.

In some embodiments, exosomes in the exosome solution have an average particle size ranging from 118.8 nm ± 1.0 nm to 156.3 nm ± 0.6 nm.

In some embodiments, a concentration of the aforementioned calcium chloride is 5 mM, and a particle size distribution of 10% to 90% (D10 to D90) of exosomes in the exosome solution obtained by the aforementioned preparation method ranges from 75.4 nm ± 1.5 nm to 171.8 nm ± 3.1 nm.

In some embodiments, a concentration of the aforementioned calcium chloride is 7.5 mM, and a particle size distribution of 10% to 90% (D 10 to D90) of exosomes in the exosome solution obtained by the aforementioned preparation method ranges from 95.3 nm ± 2.5 nm to 219.5 nm ± 5.9 nm.

In some embodiments, the aforementioned buffer is 0.1X to 2X PBS.

In some embodiments, the aforementioned buffer is phosphate buffered saline, and exosomes in the exosome solution obtained by the aforementioned preparation method have an average particle size of 167.9 nm ± 3.6 nm.

In some embodiments, the aforementioned buffer is the phosphate buffered saline, and a particle size distribution of 10% to 90% (D10 to D90) of exosomes in the exosome solution obtained by the aforementioned preparation method ranges from 103.0 nm ± 4.0 nm to 252.7 nm ± 8.0 nm.

In some embodiments, the aforementioned Tris-HCl buffer contains 0.1 M to 1 M of tris(hydroxymethyl)aminomethane hydrochloride.

In some embodiments, the aforementioned buffer is Tris-HCl buffer, and exosomes in the exosome solution obtained by the aforementioned preparation method have an average particle size of 182.0 nm ± 1.4 nm.

In some embodiments, the aforementioned buffer is Tris-HCl buffer, and a particle size distribution of 10% to 90% (D10 to D90) of exosomes in the exosome solution obtained by the aforementioned preparation method ranges from 119.6 nm ± 1.5 nm to 272.5 nm ± 8.1 nm.

In some embodiments, the aforementioned HEPES buffer contains 0.1 M to 1 M of 2-[4-(2-hydroxyethyl)-1-piperazinyl]ethanesulfonic acid.

In some embodiments, the aforementioned buffer is HEPES buffer, and most of exosomes in the exosome solution obtained by the aforementioned preparation method have an average particle size of 176.8 nm ± 2.8 nm.

In some embodiments, the aforementioned buffer is the HEPES buffer, and a particle size distribution of 10% to 90% (D10 to D90) of exosomes in the exosome solution obtained by the aforementioned preparation method ranges from 117.2 nm ± 2.9 nm to 266.3 nm ± 5.3 nm.

In some embodiments, the aforementioned exosome solution includes an epidermal growth factor (EGF), a vascular endothelial growth factor (VEGF), and a glial cell line-derived neurotrophic factor (GDNF).

In summary, the methods for preparing exosomes of any embodiment of the instant disclosure provide an exosome solution including desired exosomes in a simple environment. In some embodiments, the concentrations, average particle sizes, particle size distribution, or any combination thereof of exosomes in the resulting exosome solution may vary, depending on the types and/or concentrations of buffer used in preparation of the exosome solution. In some embodiments, the resulting exosome solution includes growth factors, such as an epidermal growth factor, a vascular endothelial growth factor, and a glial cell line-derived neurotrophic factor, and the concentrations of growth factors in the exosome solution may vary, depending on the types and/or concentrations of buffer used in preparation of the exosome solution.

### BRIEF DESCRIPTION OF THE DRAWINGS

FIG. 1 shows the steps of preparing exosomes according to an embodiment of the instant disclosure;
FIG. 2 is a bar chart showing the concentration of exosomes obtained using different concentrations of calcium ion buffer;
FIG. 3 is a bar chart showing the concentration of exosomes obtained using different buffers;
FIG. 4 is a bar chart showing the results of a cell migration experiment on exosomes obtained by different concentrations of calcium ion buffer; and
FIG. 5 is a bar chart showing the results of a cell migration experiment on exosomes obtained by different buffer.

### DETAILED DESCRIPTION

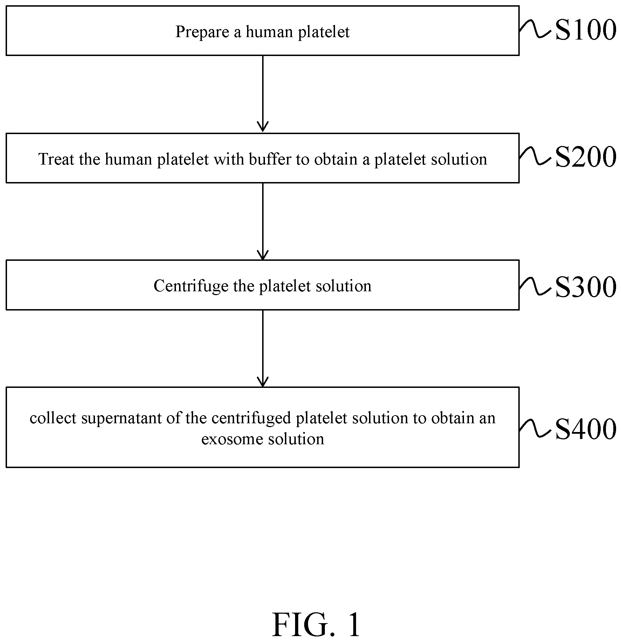

An embodiment of the instant disclosure is provided. See FIG. 1. Firstly, a human platelet is provided (designated step S100). In some embodiments of step S100, after a fresh human platelet concentrate unit is taken, plasma in the human platelet concentrate unit is removed to obtain a human platelet. By way of examples, a fresh human platelet concentrate unit is taken from a registered blood bank, and then is centrifuged at 3000 ×g for 30 min. Then supernatant is removed and precipitate is collected so as to remove plasma. Next, phosphate buffered saline filtered through a 0.02 µm sieve is added to redissolve the precipitate, and the redissolved precipitate is centrifuged at 3000 ×g for 30 min. Supernatant is removed and precipitate is collected so as to remove residual plasma, that is, a plasma-removed human platelet is obtained.

After step S100, the human platelet is treated with buffer to obtain a platelet solution (designated step S200). Here, the platelet solution has contained exosomes secreted by the human platelet under the stimulation of the buffer.

In some embodiments of step S200, after a buffer is added to the human platelet, the human platelet is suspended in the buffer to obtain a platelet solution. In some other embodiments of step S200, after adding buffer to the human platelet, the human platelet is mixed in the buffer by stirring, well shaking or the like to obtain a platelet solution. By way of examples, when the human platelet comes into contact with the buffer, the buffer would activate or react with the human platelet to form a platelet solution.

In some embodiments of step S200, the human platelet is treated with a buffer at an effective temperature to obtain a platelet solution. By way of examples, the effective temperature may be 37°C. In an example, the human platelet is treated with a buffer at 37°C to obtain a platelet solution.

In some embodiments of step S200, the human platelet is treated with buffer for an effective period of time. By way of examples, the human platelet is suspended in the buffer to form a platelet suspension, and then the platelet suspension is allowed to stand for an effective time to obtain a platelet solution. In some embodiments, the effective period of time may be from 1 h to 3 h. By way of examples, the effective period of time may be 60 min, 90 min, 120 min, 150 min, or 180 min. In an example, the human platelet is incubated in the buffer at 37°C for 1 h.

The aforementioned buffer is calcium ion buffer, phosphate buffered saline (PBS), a tris(hydroxymethyl)aminomethane hydrochloride (Tris-HCl, pH 7.5) buffer (Tris-HCl buffer, pH 7.5) or a 2-[4-(2-hydroxyethyl)-1-piperazinyl]ethanesulfonic acid (HEPES) buffer.

In some embodiments, the calcium ion buffer includes phosphate buffered saline and at least one of calcium chloride (CaCl₂), calcium carbonate (CaCO₃), and calcium gluconate (C₁₂H₂₂CaO₁₄). In some embodiments, a calcium ion concentration of the calcium ion buffer ranges from 2 mM to 20 mM. By way of examples, the calcium ion buffer is prepared from phosphate buffered saline and calcium chloride, and the calcium chloride has a concentration ranged from 2 mM to 20 mM. In some examples, the calcium ions or the calcium chloride may have a concentration of 2 mM, 3 mM, 4 mM, 5 mM, 6 mM, 7 mM, 7.5 mM, 8 mM, 9 mM, 10 mM, 11 mM, 12 mM, 13 mM, 14 mM, 15 mM, 16 mM, 17 mM, 18 mM, 19 mM, or 20 mM.

In some embodiments, the PBS is phosphate buffered saline including sodium chloride (NaCl) and potassium chloride (KCl) that can be obtained after a commercially available PBS tablet (such as a Sigma tablet) is dissolved in deionized water. By way of examples, 1X PBS can be obtained by dissolving the commercially available PBS tablet in 200 mL of deionized water, where the 1X PBS contains 137 mM of sodium chloride, 2.7 mM of potassium chloride, and 10 mM of phosphate buffer. In some embodiments, 0.1X PBS to 2X PBS, such as 0.1X PBS, 0.5X PBS, 1X PBS 1.5X PBS and 2X PBS, can be obtained by adjusting the addition amount of deionized water as needed. In some examples, the buffer is 1X PBS.

In some embodiments, the Tris-HCl buffer (pH 7.0 to 8.0) is a solution of trishydroxymethylaminomethane (Tris) dissolved in deionized water, and its pH is adjusted to 7.0 to 8.0 with hydrochloric acid (HCl). In some embodiments, the buffer used is 0.1 M to 1 M of Tris-HCl buffer (pH 7.0 to 8.0). By way of examples, a concentration of the Tris-HCl buffer (pH 7.0 to 8.0) may be 0.1 M, 0.2 M, 0.3 M, 0.4 M, 0.5 M, 0.6 M, 0.7 M, 0.8 M, 0.9 M, or 1.0 M. In some examples, the buffer is 0.1 M of Tris-HCl buffer (pH 7.5).

In some embodiments, the HEPES buffer is a solution of 2-[4-(2-hydroxyethyl)-1-piperazinyl]ethanesulfonic acid (HEPES) dissolved in redistilled water (ddH₂O), and its pH value is adjusted to 7.0 to 8.0 with sodium hydroxide (NaOH). In some embodiments, the buffer used is 0.1 M to 1 M of HEPES buffer. By way of examples, a concentration of the HEPES buffer may be 0.1 M, 0.2 M, 0.3 M, 0.4 M, 0.5 M, 0.6 M, 0.7 M, 0.8 M, 0.9 M, or 1.0 M. In some examples, the buffer is 0.1 M of HEPES buffer, and its pH value is 7.5.

After step S200, the platelet solution is centrifuged (designated step S300). By way of examples, the platelet solution obtained in step S200 is centrifuged at 3000 ×g for 30 min, and the centrifuged platelet solution is separated into supernatant and precipitate.

After step S300, supernatant of the centrifuged platelet solution is collected to obtain an exosome solution (designated step S400). By way of examples, the supernatant of the centrifuged platelet solution obtained in step S300 is collected to obtain an exosome solution.

Moreover, the platelets are treated with different buffers at different times, and the resulting exosomes vary in concentration and particle size.

In some embodiments, the resulting exosome solution is treated with a calcium ion buffer including different concentrations (such as 5 mM, 7.5 mM, 15 mM, or 20 mM) of calcium ions, and exosomes included in the exosome solution have an average exosome concentration ranged from (6200 ± 403) ×10⁷/mL to (9740 ± 252) ×10⁷/mL, and these exosomes have an average particle size ranged from 118.8 nm ± 1.0 nm to 156.3 nm ± 0.6 nm.

In some embodiments, the resulting exosome solution is treated with a calcium ion buffer including 5 mM of calcium ions, and the particle size distribution of 10% to 90% (D10 to D90) of exosomes included in the exosome solution falls between 75.4 nm ± 1.5 nm and 171.8 nm ± 3.1 nm.

In some embodiments, the resulting exosome solution is treated with a calcium ion buffer including 7.5 mM of calcium ions, and the particle size distribution of 10% to 90% (D10 to D90) of exosomes included in the exosome solution falls between 95.3 nm ± 2.5 nm and 219.5 nm ± 5.9 nm.

In some embodiments, the resulting exosome solution is treated with phosphate buffered saline, and exosomes contained in the exosome solution have an average exosome concentration of (1890 ± 34.6) ×10⁷/mL, and these exosomes have an average particle size of 167.9 nm ± 3.6 nm.

In some embodiments, the resulting exosome solution is treated with phosphate buffered saline, and the particle size distribution of 10% to 90% (D10 to D90) of exosomes contained in the exosome solution falls between 103.0 nm ± 4.0 nm and 252.7 nm ± 8.0 nm.

In some embodiments, the resulting exosome solution is treated with 0.1 M of Tris-HCl buffer, and exosomes included in the exosome solution have an average exosome concentration of (4230 ± 322) ×10⁷/mL, and these exosomes have an average particle size of 182.0 nm ± 1.4 nm.

In some embodiments, the resulting exosome solution is treated with 0.1 M of Tris-HCl buffer, and the particle size distribution of 10% to 90% (D10 to D90) of exosomes included in the exosome solution falls between 119.6 nm ± 1.5 nm and 272.5 nm ± 8.1 nm.

In some embodiments, the resulting exosome solution is treated with 0.1 M of HEPES buffer, and exosomes included in the exosome solution have an average exosome concentration of (2460 ± 178) ×10⁷/mL, and these exosomes have an average particle size of 176.8 nm ± 2.8 nm.

In some embodiments, the resulting exosome solution is treated with 0.1 M of HEPES buffer, and the particle size distribution of 10% to 90% (D10 to D90) of exosomes included in the exosome solution falls between 117.2 nm ± 2.9 nm and 266.3 nm ± 5.3 nm.

In some embodiments, the aforementioned exosome solution can promote cell migration, and has a potential to be applied to wound healing. In some embodiments, the aforementioned exosome solution can promote cell migration of epidermal cells, thereby promoting wound healing and/or repair of the skin. For studies related to migration-wound healing, refer to an article by Ayman Grada et al. (2017, Feb) J Invest Dermatol., 137(2):e11-e16, an article by Luis G Rodriguez et al. (2005) Methods Mol Biol., 294:23-9, an article by Bereiter-Hahn, J (1984) Biology of the Integument (Berlin and Heidelberg: Springer-Verlag),443-471, an article by Roberta Addis et al. (2020) Int J Med Sci, 17(8): 1030-1042, an article by Yuan Hu Xuan et al. (2014) PLoS ONE:9(9): e108182, an article by SatishPatel et al. (2019) Biomedicine & Pharmacotherapy 112: 1086, an article by R Madhyastha et al (2012) Int Wound J, 9:355-361, and an article by Marcia L.Usui (2008)Journal of Histochemistry and Cytochemistry, 56(7): 687-696.

In some embodiments, the exosome solution includes growth factors such as an epidermal growth factor (EGF), a vascular endothelial growth factor (VEGF), and a glial cell line-derived neurotrophic factor (GDNF). In some embodiments, the growth factors such as EGF, VEGF, and GDNF are encapsulated in exosomes or a buffer located outside the exosomes.

In some embodiments, the exosome solution containing EGF can promote growth of epidermal cells to cover a wound, thus it can be applied clinically to wound treatment and used as a drug, and may also have a potential to treat chronic diabetic foot ulcers. In some embodiments, the exosome solution containing VEGF can stimulate angiogenesis of granulation tissue and stimulate peripheral indirect angiogenesis, and thus it can be applied clinically to wound healing and related research on diabetic foot ulcers. In some embodiments, the exosome solution containing GDNF can promote wound healing.

For studies related to growth factor-wound healing, refer to an article by Kanchan Shakhakarmi et al. (2023) Archives of Pharmacal Research, 46:299-322, an article by J Hardwicke et al. (2008, Jun) Surgeon, 6(3): 172-7, an article by Mert Dumantepe et al. (2015, Apr) Growth Factors. 33(2): 128-32, an article by Tiaka EK, Papanas N et al. (2012, Mar) Perspect Vasc Surg Endovasc Ther.; 24(1):37-44, an article by Jorge Berlanga-Acosta et al. (2020, Jul) MEDICC Rev; 22(3):24-31, an article by Stephan Barrientos et al. (2014) Wound Repair Regen; 22(5): 569-578, an article by David O Bates et al. (2003, Jun) Int J Low Extrem Wounds; 2(2): 107-20, an article by Aakansha Giri Goswami et al. (2022, Aug) Growth Factors.; 40(3-4):73-88, an article by Mohammad Zubair & Jamal Ahmad(2019) Reviews in Endocrine and Metabolic Disorders 20: P207-217, an article by Neda Vishlaghiet al. (2022, Apr) Exp Dermatol.;31(4):577-581, an article by Thomas S Lisse et al. (2020, Jun 12)NPJ Regen Med.; 5:13, and an article by Simon Mwangi et al. (2008, Mar) Gastroenterology; 134(3):727-37.

In some embodiments, the EGF contained in the exosome solution has a concentration ranging from 20.95 ng/mL to 128 ng/mL. In some embodiments, the VEGF contained in the exosome solution has a concentration ranging from 417.88 ng/mL to 1332 ng/mL. In some embodiments, the GDNF contained in the exosome solution has a concentration ranging from 18.722 ng/mL to 184 ng/mL.

The following experimental data is represented by mean ± standard deviation (SD), and differences between two groups are analyzed with a student's t-test. In the figures, "*" represents that p value is less than 0.05, "**" represents that p value is less than 0.01, and "***" represents that p value is less than 0.001. The more the "*", the more significant the statistical differences.

### Example 1: Preparation of Exosome Solution - Calcium Ion Buffer

Firstly, a fresh human platelet concentrate unit was taken from a registered blood bank (source: Taipei Blood Donor Center), and then was centrifuged at 3000 ×g for 30 min. Then supernatant was removed and precipitate was collected so as to remove plasma. Next, 1X PBS (purchased from Gibco) filtered through a 0.02 µm sieve was added to wash the precipitate, and the washed precipitates was then centrifuged at 3000 ×g for 30 min to obtain precipitate so as to remove residual plasma, that is, a plasma-removed human platelet was obtained.

The plasma-removed human platelet was divided into five groups: a control group, an experimental group (A), an experimental group (B), an experimental group (C), and an experimental group (D), respectively. Buffer used in the control group was 1X PBS (purchased from Sigma; prepared from a Sigma tablet), buffer used in the experimental group (A) was 5 mM of calcium ion buffer prepared from the 1X PBS and calcium chloride (purchased from Spectrum), buffer used in the experimental group (B) was 7.5 mM of calcium ion buffer prepared from the 1X PBS and the calcium chloride, buffer used in the experimental group (C) was 15 mM of calcium ion buffer prepared from the 1X PBS and the calcium chloride, and a buffer used in the experimental group (D) was 20 mM of calcium ion buffer prepared from the 1X PBS and the calcium chloride.

Next, the plasma-removed human platelets in the five groups were treated with five groups of different buffers respectively at 37°C for 1 h to obtain a platelet solution of each group. The platelet solution of each group was centrifuged at 3000 ×g for 30 min, and supernatant of the platelet solution of each group was collected to obtain an exosome solution of each group.

### Example 2: Preparation of Exosome Solution - Different Types of Buffer

Firstly, a fresh human platelet concentrate unit was taken from a registered blood bank (source: Taipei Blood Donor Center), and then was centrifuged at 3000 ×g for 30 min. Then supernatant was removed and precipitate was collected so as to remove plasma. Next, 1X PBS filtered through a 0.02 µm sieve was added to wash the precipitate, and the washed precipitate was then centrifuged at 3000 ×g for 30 min to obtain precipitate so as to remove residual plasma, that is, a plasma-removed human platelet was obtained.

The plasma-removed human platelet was divided into 4 groups: a control group, an experimental group (I), an experimental group (II), and an experimental group (III), respectively. Buffer used in the control group was saline (purchased from Sigma), buffer used in the experimental group (I) was 1X PBS (purchased from Sigma; prepared with a Sigma tablet), buffer used in the experimental group (II) was 0.1 M of Tris-HCl buffer (pH 7.5; purchased from Sigma), and buffer used in the experimental group (III) was 0.1 M of HEPES buffer (purchased from Sigma).

Next, the plasma-removed human platelets in the four groups were treated with four groups of different buffers respectively at 37°C for 1 h to obtain a platelet solution of each group. The platelet solution of each group was centrifuged at 3000 ×g for 30 min, and supernatant of the platelet solution of each group was collected to obtain an exosome solution of each group.

### Example 3: Detection of Concentration of Exosomes in Exosome Solution of Each Group

Nanoparticle tracking analysis (NTA) was performed using a Nanosight instrument on the 5 groups of exosome solutions prepared in Example 1 and the 4 groups of exosome solutions obtained in Example 2 to obtain the concentration of exosomes of the exosome solution in each group.

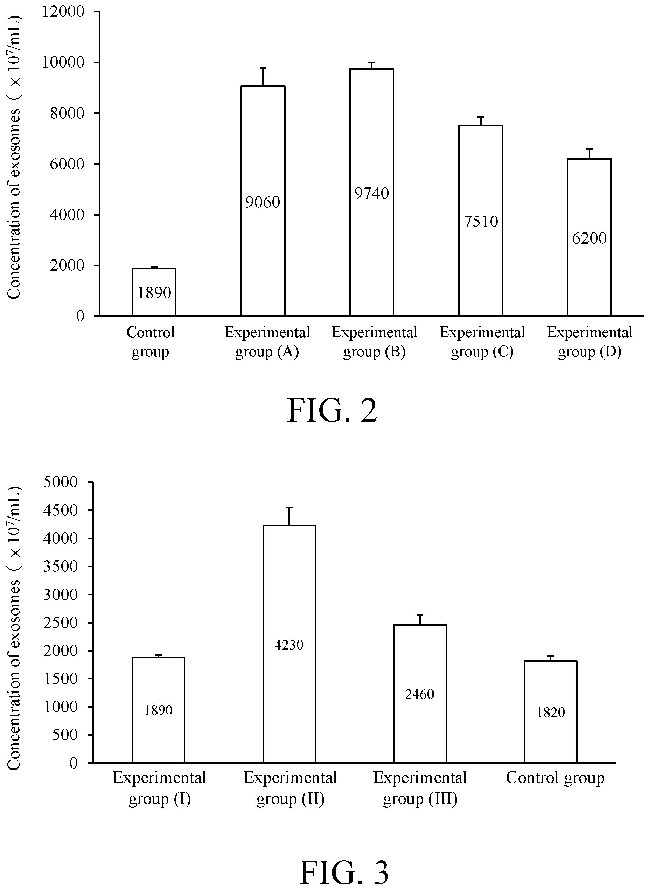

The detection results of the concentration of the exosomes in the exosome solutions in the 5 groups prepared in Example 1 are as shown in Table 1 and FIG. 2.

**Table 1**

| Group | Buffer | Average concentration (×10⁷/mL) | Standard deviation |
|---|---|---|---|
| Control group [1] | 1X PBS | 1890 | 34.6 |
| Experimental group (A) | 5 mM of calcium ion buffer | 9060 | 723 |
| Experimental group (B) | 7.5 mM of calcium ion buffer | 9740 | 252 |
| Experimental group (C) | 15 mM of calcium ion buffer | 7510 | 338 |
| Experimental group (D) | 20 mM of calcium ion buffer | 6200 | 403 |

Here, the control group of Example 1 was labeled as "control group [1]" to distinguish it from the control group of Example 2.

As can be seen from Table 1 and FIG. 2, the buffer used in the control group [1] is the 1X PBS, so after human platelet is treated with the 1X PBS, the concentration of the exosomes secreted by the human platelet under stimulation is 1890×10⁷/mL. The buffer used in the experimental group (A) was 5 mM of calcium ion buffer, so after human platelet was treated with the 5 mM of calcium ion buffer, the concentration of the exosomes secreted by the human platelet under stimulation was 9060×10⁷/mL. The buffer used in the experimental group (B) was 7.5 mM of calcium ion buffer, so after human platelet was treated with the 7.5 mM of calcium ion buffer, the concentration of the exosomes secreted by the human platelet under stimulation was 9740×10⁷/mL. The buffer used in the experimental group (C) was 15 mM of calcium ion buffer, so after human platelet was treated with the 15 mM of calcium ion buffer, the concentration of the exosomes secreted by the human platelet under stimulation was 7510×10⁷/mL. The buffer used in the experimental group (D) was 20 mM of calcium ion buffer, so after human platelet was treated with the 20 mM of calcium ion buffer, the concentration of the exosomes secreted by the human platelet under stimulation was 6200×10⁷/mL.

From this, it could be seen that the addition of calcium ions promoted the secretion of the exosomes by the human platelet, and the concentration of the exosomes in the resulting exosome solution from the human platelet treated with the 5 mM or 7.5 mM of calcium ion buffer was increased by at least 4.5 folds in comparison with that of the exosomes in the resulting exosome solution from the human platelet treated with the 1X PBS.

The detection results of the concentration of the exosomes in the exosome solutions in the 4 groups prepared in Example 2 are as shown in FIG. 3.

**Table 2**

| Group | Buffer | Average concentration (×10⁷/mL) | Standard deviation |
|---|---|---|---|
| Experimental group (I) | 1X PBS | 1890 | 34.6 |
| Experimental group (II) | 0.1 M of Tris-HCl buffer (pH 7.5) | 4230 | 322 |
| Experimental group (III) | 0.1 M of HEPES buffer | 2460 | 178 |
| Control group [2] | Saline | 1820 | 96.2 |

Here, the control group of Example 2 was labeled as "control group [2]" to distinguish it from the control group of Example 1.

As can be seen from Table 2 and FIG. 3, the buffer used in the control group [2] was saline, so after a human platelet was treated with the saline, the concentration of the exosomes secreted by the human platelet under stimulation was 1820×10⁷/mL. The buffer used in the experimental group (I) was 1X PBS, so after human platelet was treated with the 1X PBS, the concentration of the exosomes secreted by the human platelet under stimulation was 1890×10⁷/mL. The buffer used in the experimental group (II) was 0.1 M of Tris-HCl buffer (pH 7.5), so after human platelet is treated with the Tris-HCl buffer (pH 7.5), the concentration of the exosomes secreted by the human platelet under stimulation was 4230×10⁷/mL. The buffer used in the experimental group (III) was 0.1 M of HEPES buffer, so after human platelet was treated with the 0.1 M of HEPES buffer, the concentration of the exosomes secreted by the human platelet under stimulation was 2460×10⁷/mL.

It could be seen that different types of buffers promoted the secretion of the exosomes from the human platelet in comparison with the saline. Based on this, a user can choose different buffers according to a desired concentration of the exosomes to prepare an exosome solution.

### Example 4: Particle Size Information of Exosomes in Exosome Solution of Each Group

Nanoparticle tracking analysis (NTA) was performed using a Nanosight instrument on the 4 groups of exosome solutions prepared in Example 1 and the 4 groups of exosome solutions obtained in Example 2 to obtain particle size information such as the average particle size and particle size distribution of the exosome solution of each group.

The detection results of the particle size information of the exosomes in the exosome solutions in the 4 groups prepared in Example 1 are as shown in Table. 3.

**Table 3**

| Group | Experimental group (A) | Experimental group (B) | Experimental group (C) | Experimental group (D) |
|---|---|---|---|---|
| Calcium ion concentration in buffer | 5 mM | 7.5 mM | 15 mM | 20 mM |
| Average particle size (nm) | 118.8 ± 1.0 | 156.3 ± 0.6 | 149.1 ± 4.8 | 152.9 ± 3.9 |
| Mode particle size (nm) | 91.2 ± 6.2 | 128.6 ± 7.5 | 148.1 ± 6.5 | 155.0 ± 9.1 |
| D10 (nm)-D90 (nm) | 75.4 ± 1.5 - 171.8 ± 3.1 | 95.3 ± 2.5 - 219.5 ± 5.9 | 79.2 ± 8.4 - 217.9 ± 0.9 | 59.5 ± 7.2 - 221.5 ± 21.5 |

In Table 3, D10-D90 represents particle size distribution of 10% to 90%.

As can be seen from Table 3, the buffer used in the experimental group (A) was the 5 mM of calcium ion buffer, so after human platelet was treated with the 5 mM of calcium ion buffer, the average particle size, the mode particle size and the particle size distribution of 10% to 90% of the exosomes secreted by the human platelet under stimulation were 118.8 ± 1.0 nm, 91.2 ± 6.2 nm, and 75.4 ± 1.5 nm to 171.8 ± 3.1 nm, respectively. The buffer used in the experimental group (B) was 7.5 mM of calcium ion buffer, so after human platelet was treated with the 7.5 mM of calcium ion buffer, the average particle size, the mode particle size and the particle size distribution of 10% to 90% of the exosomes secreted by the human platelet under stimulation are 156.3 ± 0.6 nm, 128.6 ± 7.5 nm, and 95.3 ± 2.5 nm to 219.5 ± 5.9 nm, respectively. The buffer used in the experimental group (C) was 15 mM of calcium ion buffer, so after human platelet was treated with the 15 mM of calcium ion buffer, the average particle size, the mode particle size and the particle size distribution of 10% to 90% of the exosomes secreted by the human platelet under stimulation were 149.1 ± 4.8 nm, 148.1 ± 6.5 nm, and 79.2 ± 8.4 nm to 217.9 ± 0.9 nm, respectively. The buffer used in the experimental group (D) was 20 mM of calcium ion buffer, so after human platelet was treated with the 20 mM of calcium ion buffer, the average particle size, the mode particle size and the particle size distribution of 10% to 90% of the exosomes secreted by the human platelet under stimulation were 152.9 ± 3.9 nm, 155.0 ± 9.1 nm, and 59.5 ± 7.2 nm to 221.5 ± 21.5 nm, respectively.

That is, after the concentration of the calcium ions was adjusted, exosomes of different particle sizes could be obtained. Therefore, a user can choose different calcium ion buffers for exosomes of desired particle sizes to obtain exosome solutions including exosomes of corresponding particle sizes.

The detection results of the particle size information of the exosomes in the exosome solutions in the 4 groups prepared in Example 2 are as shown in Table. 4.

**Table 4**

| Group | Experimental group (I) | Experimental group (II) | Experimental group (III) | Control group |
|---|---|---|---|---|
| Buffer | 1X PBS | 0.1 M of Tris-HCl buffer (pH 7.5) | 0.1 M of HEPES buffer | Saline |
| Average particle size (nm) | 167.9 ± 3.6 | 182.0 ± 1.4 | 176.8 ± 2.8 | 167.2 ± 1.1 |
| Mode particle size (nm) | 142.8 ± 2.0 | 156.5 ± 4.2 | 134.4 ± 2.4 | 143.8 ± 5.4 |
| D10 (nm)- | 103.0 ± 4.0 | 119.6 ± 1.5 | 117.2 ± 2.9 | 108.9 ± 3.0 |
| D90 (nm) | 252.7 ± 8.0 | 272.5 ± 8.1 | 266.3 ± 5.3 | 242.5 ± 6.4 |

In Table 4, D10-D90 represents particle size distribution of 10% to 90%.

The buffer used in the control group was saline, so after human platelet was treated with the saline, the average particle size, the mode particle size and the particle size distribution of 10% to 90% of the exosomes secreted by the human platelet under stimulation were 167.2 ± 1.1 nm, 143.8 ± 5.4 nm, and 108.9 ± 3.0 nm to 242.5 ± 6.4 nm, respectively. The buffer used in the experimental group (I) was 1X PBS, so after a human platelet was treated with the 1X PBS, the average particle size, the mode particle size and the particle size distribution of 10% to 90% of the exosomes secreted by the human platelet under stimulation were 167.9 ± 3.6 nm, 142.8 ± 2.0 nm, and 103.0 ± 4.0 nm to 252.7 ± 8.0 nm, respectively. The buffer used in the experimental group (II) was 0.1 M of Tris-HCl buffer (pH 7.5), so after human platelet was treated with the Tris-HCl buffer (pH 7.5), the average particle size, the mode particle size and the particle size distribution of 10% to 90% of the exosomes secreted by the human platelet under stimulation were 182.0 ± 1.4 nm, 156.5 ± 4.2 nm, and 119.6 ± 1.5 nm to 272.5 ± 8.1 nm, respectively. The buffer used in the experimental group (III) was 0.1 M of HEPES buffer, so after human platelet was treated with the 0.1 M of HEPES buffer, the average particle size, the mode particle size and the particle size distribution of 10% to 90% of the exosomes secreted by the human platelet under stimulation were 176.8 ± 2.8 nm, 134.4 ± 2.4 nm, and 117.2 ± 2.9 nm to 266.3 ± 5.3 nm, respectively.

That is, exosomes of different particle sizes can be obtained using different types of buffer. Therefore, a user can choose different types of buffer for exosomes of desired particle sizes to obtain exosome solutions including exosomes of corresponding particle sizes.

### Example 5: Cell Migration Experiment - Calcium Ion Buffer

Damage causes loss of cells and tissues from the body, and the body needs to repair the damage. There are two different forms of repair: regeneration and fibrous repair, both of which involve cell migration. Therefore, the design of this experiment is to observe whether a sample has the ability to promote cell migration, and then judge whether it has a potential to repair/heal a wound.

Here, cells used were normal human dermal fibroblasts (purchased from Lonza). A cell medium used was a fibroblast growth medium-2 (FGM2; purchased from Lonza). A working medium used was a 5X DMEM medium (purchased from Gibco) containing 0.1% of fetal bovine serum (FBS). Samples used were the exosome solutions prepared in Example 1 after treatment with different calcium ion buffers, and were divided into a total of 5 groups, namely, a control group, and experimental groups (A) to (D). An experimental instrument used was a 96-well ChemoTx^{®} system apparatus, which includes a lid, a filter membrane, and a microplate. An analytical instrument used was an automatic cell imaging system (ImageXpress Micro XLS).

Firstly, the normal human dermal fibroblasts were activated for later use. Moreover, the 5 groups of exosome solutions were diluted with DPBS (source: Gibco) until the concentration of the exosomes was 1.75×10¹⁰/mL, and the diluted exosome solutions were then diluted with the working medium until the concentration of the exosomes was 1.4×10¹⁰/mL to serve as samples to be detected for subsequent experiments.

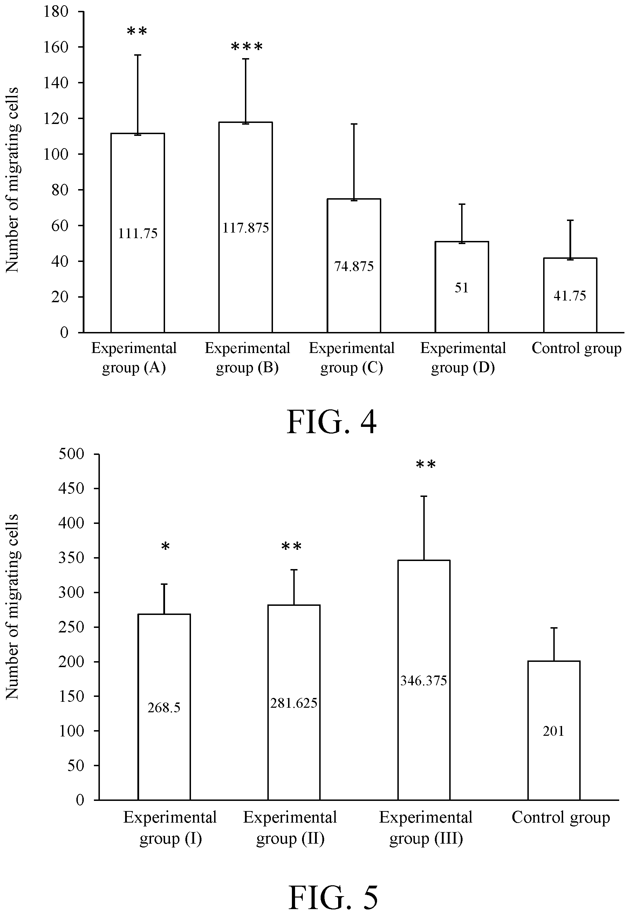

The sample to be detected in each group was injected into a 96-well microplate. The filter membrane was placed on the 96-well microplate, and the treated normal human dermal fibroblasts were added to the corresponding filter membranes of each group at a density of 1500 cells per well. Through chemotaxis of the normal human dermal fibroblasts to the samples to be detected, the normal human dermal fibroblasts would be attracted and passed through the 10 µm of filter membrane, and were moved to a position below the filter membrane. Next, culture was performed in a CO₂ incubator for 5 h. Normal human dermal fibroblasts located above in each group that did not pass through the filter membrane are removed by wiping, and then fixation was performed with methanol (source: Merck), and the normal human dermal fibroblasts were stained with a DAPI (4',6-diamidino-2-phenylindole) stain (source: Calbiochem). Finally, each group was analyzed with the ImageXpress Micro XLS instrument, and the results are as shown in FIG. 4 and Table 5. In FIG. 4, the p values are relative to those of the control group.

**Table 5**

| Group | Buffer | Average number of migrating cells | Standard deviation | P value | Rate relative to control group |
|---|---|---|---|---|---|
| Control group | 1X PBS | 41.75 | 21.2855 | 1 | 1 |
| Experimental group (A) | 5 mM of calcium ion buffer | 111.75 | 43.7844 | 0.00115 | 2.68 |
| Experimental group (B) | 7.5 mM of calcium ion buffer | 117.875 | 35.5063 | 0.00013 | 2.82 |
| Experimental group (C) | 15 mM of calcium ion buffer | 74.875 | 42.0695 | 0.06683 | 1.79 |
| Experimental group (D) | 20 mM of calcium ion buffer | 51 | 20.9626 | 0.39594 | 1.22 |

See FIG. 4 and Table 5. The average number of migrating cells of the control group was 41.75 ±± 21.2855, while the average number of migrating cells of the experimental group (A) was 111.75 ± 43.7844, the average number of migrating cells of the experimental group (B) was 117.875 ± 35.5063, the average number of migrating cells of the experimental group (C) was 74.875 ± 42.0695, and the average number of migrating cells of the experimental group (D) was 51 ± 20.9626. From the above results, it could be seen that when the buffer contain calcium ions, the prepared exosome solution increased the number of migrating cells. When the concentration of the calcium ions was 5 mM or 7.5 mM, the number of the migrating cells was increased by at least 2 folds in comparison with that of the control group. Moreover, when the concentration of the calcium ions was 15 mM, the number of the migrating cells was increased by at least 1.5 folds in comparison with that of the control group.

Based on this, the exosome solution prepared using the calcium ion buffer promoted cell migration, and had a potential to promote wound healing.

### Example 6: Cell Migration Experiment - Different Buffers

As in Example 5, the design of this experiment is to observe whether a sample has the ability to promote cell migration, and then judge whether it has a potential to repair/heal a wound.

Here, cells used were normal human dermal fibroblasts (purchased from Lonza). A cell medium used was a fibroblast growth medium-2 (FGM2; purchased from Lonza). A working medium used was a 5X DMEM medium (purchased from Gibco) containing 0.1% of fetal bovine serum (FBS). Samples used were the exosome solutions prepared in Example 1 after treatment with different calcium ion buffers, and were divided into a total of 4 groups, namely, a control group, and experimental groups (I) to (III). An experimental instrument used was a 96-well ChemoTx^{®} system apparatus, which includes a lid, a filter membrane, and a microplate. An analytical instrument used was an automatic cell imaging system (ImageXpress Micro XLS).

Firstly, the normal human dermal fibroblasts were activated for later use. Moreover, the 5 groups of exosome solutions were diluted with DPBS (source: Gibco) until the concentration of the exosomes was 1.75×10¹⁰/mL, and the diluted exosome solutions were then diluted with the working medium until the concentration of the exosomes was 1.4×10¹⁰/mL to serve as samples to be detected for subsequent experiments.

The sample to be detected in each group was injected into a 96-well microplate. The filter membrane was placed on the 96-well microplate, and the treated normal human dermal fibroblasts were added to the corresponding filter membranes of each group at a density of 5000 cells per well. Through chemotaxis of the normal human dermal fibroblasts to the samples to be detected, the normal human dermal fibroblasts would be attracted and passed through the 10 µm of filter membrane, and were moved to a position below the filter membrane. Next, culture was performed in a CO₂ incubator for 5 h. Normal human dermal fibroblasts located above in each group that did not pass through the filter membrane are removed by wiping, and then fixation was performed with methanol (source: Merck), and the normal human dermal fibroblasts were stained with a DAPI (4',6-diamidino-2-phenylindole) stain (source: Calbiochem). Finally, each group was analyzed with the ImageXpress Micro XLS instrument, and the results are as shown in FIG. 5 and Table 6. In FIG. 5, the p values are relative to those of the control group.

**Table 6**

| Group | Buffer | Average number of migrating cells | Standard deviation | P value |
|---|---|---|---|---|
| Control group | Saline | 201 | 47.91063 | 1 |
| Experimental group (I) | 1X PBS | 268.5 | 43.52011 | 0.010553 |
| Experimental group (II) | 0.1 M of Tris-HCl buffer (pH 7.5) | 281.625 | 50.93957 | 0.005687 |
| Experimental group (III) | 0.1 M of HEPES buffer | 346.375 | 92.24956 | 0.001436 |

See FIG. 5 and Table 6. The average number of migrating cells of the control group was 201 ± 47.91063, while the average number of migrating cells of the experimental group (I) was 268.5 ± 43.52011, the average number of migrating cells of the experimental group (II) was 281.625 ± 50.93957, and the average number of migrating cells of the experimental group (III) was 346.375±92.24956. From the above results, it could be seen that when the exosome solution was prepared using different buffers, the exosome solution had the ability to increase the number of migrating cells. When the buffer was 0.1 M of HEPES buffer, the number of the migrating cells was increased by at least 1.5 folds in comparison with that of the control group. Based on this, the exosome solution prepared using different buffers promoted cell migration, and had a potential to promote wound healing.

### Example 7: Growth Factor Analysis

Here, exosome solutions in a total of 3 groups, namely the control group, the experimental group (A), and the experimental group B in Example 1, and exosome solutions in a total of 4 groups, namely the control group, the experimental group (I), the experimental group (II) and the experimental group (III) in example 2 were entrusted to RayBiotech to analyze growth factors contained in the solutions under the detection items of human growth factor array Q1 (#QAH-GF-1-SERV). The analysis results are as shown in Tables 7 and 8.

The analysis results of the growth factors of the exosome solutions in a total of 3 groups, namely the control group, the experimental group (A), and the experimental group B in Example 1 are as shown in Table 7:

**Table 7**

| Growth factors (GFs) | Full name | Control group [1] (pg/ml) | Experimental group (A) (pg/ml) | Experimental group (B) (pg/ml) |
|---|---|---|---|---|
| | | 1X PBS | 5 mM of calcium ion buffer | 7.5 mM of calcium ion buffer |
| EGF | Epidermal Growth Factor | 20.95 | 128 | 74 |
| VEGF | Vascular Endothelial Growth Factor | 974.33 | 816 | 1332 |
| GDNF | Glial Cell line-Derived Neurotrophic Factor | 18.72 | 102 | 184 |

Here, the control group of Example 1 was labeled as "control group [1]" to distinguish it from the control group of Example 2.

As can be seen from Table 7, the growth factors detected in the control group [1], the experimental group (A) and the experimental group (B) each included EGF, VEGF, and GDNF. The EGF detectable concentration, the VEGF detectable concentration, and the GDNF detectable concentration in the control group [1] were 20.95 pg/ml, 974.33 pg/ml, and 18.72 pg/ml, respectively. The EGF detectable concentration, the VEGF detectable concentration, and the GDNF detectable concentration in the experimental group (A) were 128 pg/ml, 816 pg/ml, and 102 pg/ml, respectively. The EGF detectable concentration, the VEGF detectable concentration, and the GDNF detectable concentration in the experimental group (B) were 74 pg/ml, 1332 pg/ml, and 184 pg/ml, respectively. From this, it could be seen that the concentrations of EGF and GDNF contained in the exosome solution prepared with the 5 mM or 7.5 mM of calcium ion buffer were each higher than those measured in the control group [1], and the concentration of VEGF contained in the exosome solution prepared with the 7.5 mM of calcium ion buffer was also higher than that of the control group [1]. In other words, exosome solutions prepared with different concentrations of calcium ion buffer contain different concentrations and constitutions of growth factors. Moreover, EGF, VEGF, and GDNF were each a growth factor beneficial to wound healing, so the exosome solutions prepared with different concentrations of calcium ion buffers had a potential to be applied to wound healing.

The analysis results of the growth factors of the exosome solutions in a total of 4 groups, namely the control group, the experimental group (I), the experimental group (II), and the experimental group (III) in Example 2 are as shown in Table 8:

**Table 8**

| Growth factors (GFs) | Full name | Experimental group (I) (pg/ml) 1X PBS | Experimental group (II) (pg/ml) 0.1 M of Tris-HCl buffer (pH 7.5) | Experimental group (III) (pg/ml) 0.1 M of HEPES buffer | Control group [2] (pg/ml) Saline |
|---|---|---|---|---|---|
| EGF | Epidermal Growth Factor | 20.95 | 21.47 | 29.84 | 12.17 |
| VEGF | Vascular Endothelial Growth Factor | 974.33 | 794.75 | 417.88 | 108.61 |
| GDNF | Glial Cell line-Derived Neurotrophic Factor | 18.72 | 81.7 | 72.55 | NA |

Here, the control group of Example 2 was labeled as "control group [2]" to distinguish it from the control group of Example 1.

As can be seen from Table 8, the growth factors detected in the control group [2], the experimental group (I), the experimental group (II), and the experimental group (III) each include EGF, VEGF, and GDNF. The EGF detectable concentration and the VEGF detectable concentration in the control group [2] were 12.17 pg/ml, and 108.61 pg/ml, respectively, and the concentration of GDNF was not detected. The EGF detectable concentration, the VEGF detectable concentration, and the GDNF detectable concentration in the experimental group (I) were 20.95 pg/ml, 974.33 pg/ml, and 18.72 pg/ml, respectively. The EGF detectable concentration, the VEGF detectable concentration, and the GDNF detectable concentration in the experimental group (II) were 21.47 pg/ml, 794.75 pg/ml, and 81.7 pg/ml, respectively. The EGF detectable concentration, the VEGF detectable concentration, and the GDNF detectable concentration in the experimental group (III) were 29.84 pg/ml, 417.88 pg/ml, and 72.55 pg/ml, respectively. From this, it could be seen that the concentrations of EGF and GDNF in the experimental groups (I) to (III) were higher than those of the control group, and the concentration of VEGF in the experimental group (I) was higher than those of the other three groups, the concentration of GNDF in the experimental group (II) was higher than those of the other three groups, and the concentration of EGF in the experimental group (III) was higher than those of the other three groups. In other words, the highest concentration of growth factors varied in different groups, so the constitutions of the growth factors contained also varied. Moreover, the exosome solutions of the experimental groups (I) to (III) each had the potential to be applied to wound healing.

In summary, according to the method for preparing the exosomes of any embodiment of the instant disclosure, an exosome solution including desired exosomes are provided in a simple environment, thereby avoiding variables caused by undesired components (such as growth factors from a culture solution) when the resulting exosomes are used in other experiments or applications. In some embodiments, the concentrations, average particle sizes, particle size distribution, or any combination thereof of exosomes in the resulting exosome solution may vary, depending on the types and/or concentrations of buffers used in preparation of the exosome solution.

Although the instant disclosure has been described in considerable detail with reference to certain preferred embodiments thereof, the disclosure is not for limiting the scope of the invention. Persons having ordinary skill in the art may make various modifications and changes without departing from the scope and spirit of the invention. Therefore, the scope of the appended claims should not be limited to the description of the preferred embodiments described above.

## Claims

1. A method for preparing exosomes from a human platelet, comprising:
providing a human platelet;
treating the human platelet with a buffer to obtain a platelet solution, wherein the buffer is calcium ion buffer, phosphate buffered saline (PBS), tris(hydroxymethyl)aminomethane hydrochloride (Tris-HCl) buffer, or 2-[4-(2-hydroxyethyl)-1-piperazinyl]ethanesulfonic acid (HEPES) buffer;
centrifuging the platelet solution; and
collecting supernatant of the centrifuged platelet solution to obtain an exosome solution.

2. The method according to claim 1, wherein the calcium ion buffer comprises phosphate buffered saline and at least one of calcium chloride, calcium carbonate, and calcium gluconate.

3. The method according to claim 2, wherein a concentration of the calcium chloride ranges from 2 mM to 20 mM.

4. The method according to claim 3, wherein exosomes in the exosome solution have an average particle size ranging from 118.8 nm ± 1.0 nm to 156.3 nm ± 0.6 nm.

5. The method according to claim 2, wherein a concentration of the calcium chloride is 5 mM, and a particle size distribution of 10% to 90% of exosomes in the exosome solution ranges from 75.4 nm ± 1.5 nm to 171.8 nm ± 3.1 nm.

6. The method according to claim 2, wherein a concentration of the calcium chloride is 7.5 mM, and a particle size distribution of 10% to 90% of exosomes in the exosome solution ranges from 95.3 nm ± 2.5 nm to 219.5 nm ± 5.9 nm.

7. The method according to claim 1, wherein the buffer is 0.1X to 2X PBS.

8. The method according to claim 1, wherein the buffer is the PBS, and exosomes in the exosome solution have an average particle size of 167.9 nm ± 3.6 nm.

9. The method according to claim 1, wherein the buffer is the PBS, and a particle size distribution of 10% to 90% of exosomes in the exosome solution ranges from 103.0 nm ± 4.0 nm to 252.7 nm ± 8.0 nm.

10. The method according to claim 1, wherein the Tris-HCl buffer contains 0.1 M to 1 M of tris(hydroxymethyl)aminomethane hydrochloride.

11. The method according to claim 1, wherein the buffer is the Tris-HCl buffer, and exosomes in the exosome solution have an average particle size of 182.0 nm ± 1.4 nm.

12. The method according to claim 1, wherein the buffer is the Tris-HCl buffer, and a particle size distribution of 10% to 90% of exosomes in the exosome solution ranges from 119.6 nm ± 1.5 nm to 272.5 nm ± 8.1 nm.

13. The method according to claim 1, wherein the HEPES buffer contains 0.1 M to 1 M of 2-[4-(2-hydroxyethyl)-1-piperazinyl]ethanesulfonic acid.

14. The method according to claim 1, wherein the buffer is the HEPES buffer, and exosomes in the exosome solution have an average particle size of 176.8 nm ± 2.8 nm.

15. The method according to claim 1, wherein the buffer is the HEPES buffer, and a particle size distribution of 10% to 90% of exosomes in the exosome solution ranges from 117.2 nm ± 2.9 nm to 266.3 nm ± 5.3 nm.

16. The method according to claim 1, wherein the exosome solution comprises an epidermal growth factor (EGF), a vascular endothelial growth factor (VEGF), and a glial cell line-derived neurotrophic factor (GDNF).
